# EUROPÄISCHE PATENTSCHRIFT

(11) **EP 0 613 952 B1**
(45) Veröffentlichungstag und Bekanntmachung des Hinweises auf die Patenterteilung: **15.09.1999**
(21) Anmeldenummer: 94100870.8
(22) Anmeldetag: 21.01.1994
(51) Int. Cl.: C12Q 1/00, G01N 27/327

(54) **Sensorarray**
Sensor array
Matrice de capteurs

(30) Priorität: 01.03.1993 CH 60793
(43) Veröffentlichungstag der Anmeldung: 07.09.1994
(73) Patentinhaber: Disetronic Licensing AG, 3400 Burgdorf (CH)
(72) Erfinder: Michel, Peter, CH-3400 Burgdorf (CH)

(56) Entgegenhaltungen:
- EP-A- 0 122 420
- EP-A- 0 299 778
- WO-A-92/14836
- US-A- 4 963 245
- JOURNAL OF THE CHEMICAL SOCIETY. FARADAY TRANSACTIONS, Bd.89, Nr.2, Januar 1993, CAMBRIDGE GB Seiten 361 - 367 LINDNER E ET AL : 'Flexible (Kapton based) microsensor arrays of high stability for cardiovascular applications'

## Beschreibung

Die Erfindung bezieht sich auf einen Sensor zur selektiven Feststellung oder Messung mindestens einer Stoffkomponente in einer wässerigen Lösung gemäss der Gattung des Patentanspruchs 1.

Sensoren dieser Gattung werden in grosser Zahl in der chemischen Technologie, in der Analytik und in neuerer Zeit auch in der Medizin verwendet. Insbesondere in der Medizin besteht ein grosser Bedarf an solchen sogenannten Biosensoren. Am verbreitetsten sind Biosensoren in der Diabetes-Therapie. Beispiele dafür sind in den US Patenten 4,545,382 und 4,711,245 HIGGINS enthalten. Sie bestehen im wesentlichen aus in ein Messgerät (Glucometer) einsteckbaren und nach Gebrauch wegwerfbaren Glucosensoren, auf denen das zu bestimmende Blut in kleiner Menge aufgebracht wird. Das dazu notwendige Blut entnimmt sich der Benutzer in herkömmlicher Weise durch Stechen in die Fingerkuppe. Beim Einschieben des Glucosensors in das Messgerät, wird dessen Biomembrane mit dem Messgerät elektrisch kontaktiert, so dass die durch die unterschiedlichen Glukosegehalte hervorgerufenen Spannungs- und/oder Stromänderungen der aktiven Biomembrane des Sensors an das Messgerät weitergeleitet werden. Weitere Details solcher bekannten Glucometer und Glucosensoren sind in der W092/14836 GRÄTZEL ET AL. beschrieben.

Die beiden Schriften EP-A-0 299 778 und EP-A-0 122 420 zeigen den Gebrauch eines Standardelektrolyten als Eichflüssigkeit bzw. zur Nullpunktabweichung von Elektroden eines Sensors. Aus dem US Patent 4,963,245 ist ein Sensor, der aus mehreren zeilen- oder matrix-artig angeordneten Einzelmesskammern bzw. Sensormembranen besteht, bekannt.

Nachteilig bei allen bekannten Sensoren dieser Gattung ist ihre kurze Lebensdauer und die relativ teure Herstellung. Insbesondere hat sich gezeigt, dass die Biomembrane von Glukosesensoren, deren aktiver Bestandteil aus einem instabilen Enzym, beispielsweise Glukoseoxidase, besteht, einerseits eine schwierig reproduzierbare Herstellung aufweist und anderseits verschiedenen alterungsbedingten Veränderung ihrer Eigenschaften unterworfen ist, welche ihre Lebensdauer stark beeinträchtigen. Beispiele für solche Enzyme, sowie Kombinationen davon mit einem Mediator sind in den Dokumenten WO92/14836 und WO92/14741 GRÄTZEL ET AL. beschrieben.

Hier will die Erfindung Abhilfe schaffen. Der Erfindung liegt die Aufgabe zugrunde, einen gattungsgemässen Sensor zu schaffen, der insgesamt eine verlängerte Lebensdauer aufweist bei gleichzeitig wesentlich geringeren Gesamtkosten für die Herstellung.

Die Erfindung löst die gestellte Aufgabe mit einem Sensor, welcher die Merkmale des Anspruchs 1 aufweist.

Statt dem Patienten ein Paket von mehreren hundert wegwerfbaren Einzelsensoren abzugeben, kann nun ein einziger Sensor mit einer Vielzahl von auf einem flächigen Träger, zeilen- oder matrix-artig angeordneter Einzelmembranen verwendet werden.

Die durch die Erfindung erreichten Vorteile sind im wesentlichen darin zu sehen, dass dank des erfindungsgemässen Sensors eine sehr kostengünstige Herstellung ermöglicht wird und die Lebensdauer insgesamt verlängert wird.

Die Erfindung und Weiterbildungen der Erfindung werden im folgenden anhand der teilweise schematischen Darstellungen mehrerer Ausführungsbeispiele noch näher erläutert.
Fig. 1 stellt eine perspektivische Ansicht eines Sensors in Form eines zeilenförmigen Messstreifens dar;
Fig. 2 stellt einen Querschnitt durch den Sensor nach Fig. 1 dar;
Fig. 3 stellt eine schematische Ansicht eines Messgerätes mit eingestecktem Sensor nach Fig. 1 dar;
Fig. 4 stellt einen Querschnitt durch einen modifizierten Sensor dar;
Fig. 5 stellt einen Querschnitt durch einen erfindungsgemässen Sensor dar;
Fig. 6 stellt einen Querschnitt durch eine bevorzugte Ausführungsform des erfindungsgemässen Sensor dar; und
Fig. 7 stellt eine perspektivische Ansicht eines Sensors in Form einer mehrzeiligen Matrix dar;

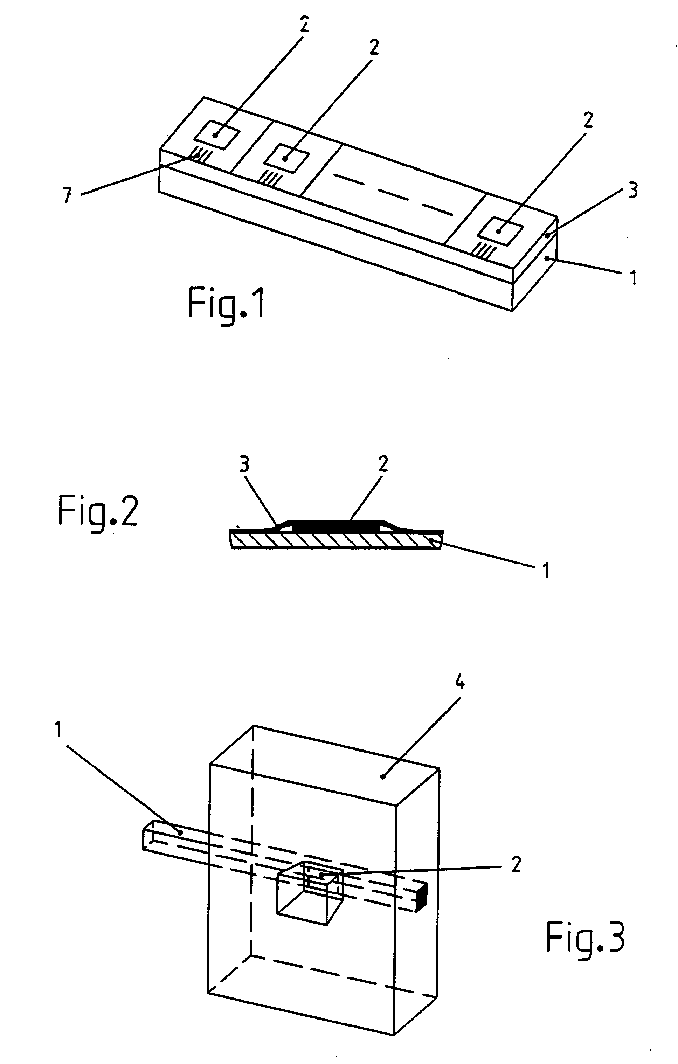

Der erfindungsgemässe Sensor weist ein Saugfliess zur Optimierung der Verteilung der zu messenden wässerigen Lösung auf. Die Figuren 1-4 und 7 zeigen kein Saugfliess, es handelt sich um allgemeine Anschauungsbeispiele für Sensoren, welche zusätzlich mit einem Saugfliess ausgestattet werden könnten bzw. erklären das Zusammenspiel zwischen Sensoren und Messgerät.

Der in Fig. 1 und 2 dargestellte Sensor besteht im wesentlichen aus einem länglichen, flächigen Träger 1 aus Kunststoff (z.B. aus Polyvinylchlorid PVC), auf welchem mehrere in einer Zeile angeordnete Einzelmembranen 2 angebracht sind, welche durch eine entfernbare Schutzschicht 3 aus Aluminiumfolie gegen äussere Einflüsse abgedeckt sind. Die Einzelmembrane 2 besteht im wesentlichen aus einer für die zu detektierende Stoffkomponente empfindlichen Enzym/Mediator-Kombination, welche über seitlich am Längsträger 1 angebrachte Elektroden 7 mit einem Datenerfassungs- oder Anzeigegerät 4 (Fig. 3) verbunden werden können. Die Elektroden 7 bestehen im Prinzip aus drei gegeneinander isolierten Elektroden, einer Arbeits- einer Kontroll- und einer Referenzelektrode, wobei letztere vorzugsweise aus Silber besteht.

Um eine Einzelmembran 2 zu aktivieren genügt es manuell den darüber liegenden Teil der Schutzschicht zu entfernen (z.B. durch Abreissen, Durchstechen oder Ausstanzen) und die zu messende wässerige Lösung, z.B. einen Bluttropfen darauf aufzubringen.

Der Sensor mit zeilenförmig angeordneten Einzelmembranen 2 kann dann auf bekannte Weise in das in Fig. 3 schematisch angedeutete Datenerfassungs- und Anzeigegerät 4 gesteckt werden. Die Sensoraktivierung kann auf verschiedene Arten erfolgen. Eine Möglichkeit besteht darin, den Sensor so weit in das Datenerfassungs- und Anzeigegerät 4 zu schieben, bis die ausgewählte Einzelmembrane 2 mit ihren Elektroden 7 in Kontakt mit der (zeichnerisch nicht dargestellten) Sensorelektronik des Datenerfassungs- und Anzeigegerät 4 kommt. Eine andere Möglichkeit, insbesondere bei in den Körper des Patienten implantierten Sensoren, besteht darin, die nächstfolgende zu aktivierende Einzelmembrane 2 durch die Sensorelektronik des Datenerfassungs- und Anzeigegerätes 4 selbst ansteuern zu lassen. Eine genauere Beschreibung des Datenerfassungs- und Anzeigegerätes 4, sowie des Aufbaus der Einzelmembranen 2 ist in der CH-PS 677.149 MICHEL enthalten.

Verbrauchte Einzelmembranen 2 können bei extrakorporalen Sensoren einfach abgebrochen und weggeworfen werden.

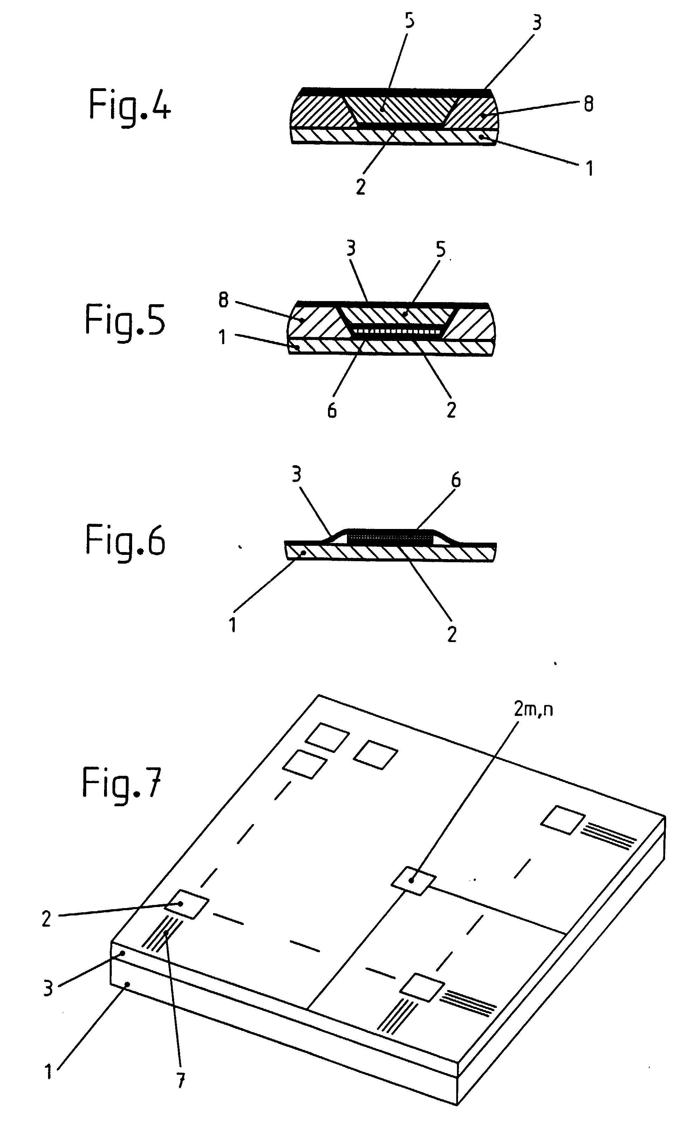

In Fig. 4 ist ein etwas komplexerer Sensor dargestellt, bei welchem auf einem länglichen, flächigen Träger 1 mit Trägerschicht 8 aus Kunststoff wiederum mehrere in einer Zeile angeordnete Einzelmembranen 2 mit einer darüberliegenden Eichflüssigkeit 5 eingelassen sind, welche durch eine entfernbare Schutzschicht 3 aus Aluminium gegen äussere Einflüsse abgedeckt sind. Die aus einer neutralen Salzlösung bestehende Eichflüssigkeit 5 erfüllt dabei mehrere Funktionen. Sie ermöglicht erstens eine Lagerung der Einzelmembranen 2 in definierter Umgebung und zweitens eine Null- oder Ein-Punkt-Eichung während des Aufstartens des Datenerfassungs- und Anzeigegerätes 4. Die dritte, für die Erfindung vorteilhafte Funktion besteht darin, dass durch Aufheizen der Eichflüssigkeit 5 - analog zu einem Tintenstrahldrucker - die Schutzschicht 3 durch innere Energie aufgesprengt werden kann um die Einzelmembranen 2 für den Messvorgang zu aktivieren.

Aus konstruktiven Gründen ist bei der Ausführung mit der Eichlösung 5 gemäss Fig. 4 auf dem Träger 1 eine weitere Trägerschicht 8 aus Kunststoff angeordnet, auf welche dann die Schutzschicht 3 zu liegen kommt.

In Fig. 5 ist ein erfindungsgemässer Sensor in Anlehnung an Fig. 4 dargestellt, bei welchem zwischen der Einzelmembrane 2 und der Eichflüssigkeit 5 ein Saugfliess 6 angebracht ist, welches die Verteilung der zu messenden wässerigen Lösung optimiert. Als Saugfliess 6 eignet sich beispielsweise ein Nylonnetz.

In Fig. 6 ist eine bevorzugte Ausführung des erfindungsgemässen Sensors dargestellt, bei welchem auf dem Träger 1 die Einzelmembranen 2 mit einem darüber liegenden Saugfliess 6 aufgebracht und mit der Schutzfolie 3 abgedeckt sind.

In Fig. 7 ist ein Sensor in Form einer mehrzeiligen Matrix von einzeln angeordneten Einzelmembranen 2_{m.n} (Einzelmembrane in der m.ten Zeile und n.ten Spalte) dargestellt. Diese Form eignet sich besonders für implantierbare Sensoren, bei welchen die zu aktivierende Einzelmembran 2 direkt von der vorhandenen Elektronik des Datenerfassungs- und Anzeigegerätes 4 angesteuert wird und die Eichflüssigkeit 5 zum Sieden bringt.

## Patentansprüche

1. Sensor zur selektiven Feststellung oder Messung mindestens einer Stoffkomponente in einer wässerigen Lösung, mit mindestens zwei elektrisch leitenden, voneinander isolierten Elektroden (7), welche an ein Datenerfassungsgerät (4) anschliessbar sind, und einer für die zu detektierende Stoffkomponente empfindlichen Sensormembrane, welche auf einem flächigen Träger (1) in Form mehrerer zeilen- oder matrix-artig angeordneter Einzelmembranen (2) ausgebildet ist, dadurch gekennzeichnet, dass auf den Einzelmembranen Mittel, in Form eines Saugfliesses (6), zur Optimierung der Verteilung der zu messenden wässerigen Lösung angeordnet sind.

2. Sensor nach Anspruch 1, dadurch gekennzeichnet, dass die Einzelmembranen (2) mit einer entfernbaren Schutzschicht (3) gegen äussere Einflüsse, vorzugsweise einer Metallfolie, abgedeckt sind.

3. Sensor nach Anspruch 1 oder 2, dadurch gekennzeichnet, dass das Saugfliess (6) ein Nylonnetz ist.

4. Sensor nach einem der Ansprüche 1 bis 3, dadurch gekennzeichnet, dass zwischen den Einzelmembranen (2), bzw. den darauf angeordneten Mitteln zur Optimierung der Verteilung der zu messenden wässerigen Lösung, und der Schutzschicht (3) eine Eichflüssigkeit (5), vorzugsweise eine neutrale Salzlösung vorgesehen ist.

5. Sensor nach einem der Ansprüche 1 bis 4, dadurch gekennzeichnet, dass die Einzelmembranen (2) ein Enzym enthalten, welches auf die zu detektierende Stoffkomponente empfindlich ist.

6. Sensor nach einem der Ansprüche 1 bis 5, dadurch gekennzeichnet, dass die Einzelmembranen (2) zusätzlich zum Enzym einen Mediator enthalten.

7. Sensor nach einem der Ansprüche 1 bis 6, dadurch gekennzeichnet, dass die Einzelmembranen (2) auf physiologische Stoffe, vorzugsweise Glukose, in der Körperflüssigkeit empfindlich sind.

8. Sensor nach einem der Ansprüche 1 bis 7, dadurch gekennzeichnet, dass die Elektroden (7) aus drei gegeneinander isolierten Elektroden bestehen, einer Arbeits- einer Kontroll- und einer Referenzelektrode, wobei letztere vorzugsweise aus Silber besteht.

## Claims

1. A sensor for selectively detecting or measuring at least one substance component in an aqueous solution, having at least two electrically conducting electrodes (7) which are insulated from each other and which are connectable to a data acquisition device (4). and a sensor membrane which is sensitive for the substance component to be detected and which is provided on a flat carrier (1) in the form of a plurality of individual membranes (2) arranged in a line-like or matrix-like configuration, characterised in that arranged on the individual membranes are means, in the form of an absorption fleece (6), for optimising the distribution of the aqueous solution to be measured.

2. A sensor according to claim 1 characterised in that the individual membranes (2) are covered over by a removable protective layer (3) for protection from external influences, preferably a metal foil.

3. A sensor according to claim 1 or claim 2 characterised in that the absorption fleece (6) is a nylon mesh.

4. A sensor according to one of claims 1 to 3 characterised in that a calibration fluid (5). preferably a neutral salt solution, is provided between the individual membranes (2) or the means arranged thereon for optimising the distribution of the aqueous solution to be measured, and the protective layer (3).

5. A sensor according to one of claims 1 to 4 characterised in that the individual membranes (2) contain an enzyme which is sensitive to the component to be detected.

6. A sensor according to one of claims 1 to 5 characterised in that the individual membranes (2) contain a mediator in addition to the enzyme.

7. A sensor according to one of claims 1 to 6 characterised in that the individual membranes (2) are sensitive to physiological substances, preferably glucpse, in the body fluid.

8. A sensor according to one of claims 1 to 7 characterised in that the electrodes (7) comprise three electrodes which are insulated from each other, a working electrode, a control electrode and a reference electrode, the latter preferably comprising silver.

## Revendications

1. Capteur pour la détermination sélective ou la mesure d'au moins un composant de matière dans une solution aqueuse, avec au moins deux électrodes électriquement conductrices (7), isolées entre elles qui peuvent être connectées à un appareil de saisie de données (4), et avec une membrane de capteur sensible pour les composants de matière à détecter, membrane qui est formée sur un support plat (1) à partir de plusieurs membranes individuelles disposées par lignes ou sous de matrice (2), caractérisé en ce que sur les membranes individuelles sont disposés des moyens sous forme d'un tapis aspirant (6) pour optimiser la répartition de la solution aqueuse à mesurer.

2. Capteur selon la revendication 1, caractérisé en ce que les membranes individuelles (2) sont revêtues d'une couche de protection amovible (3) contre les influences externes, de préférence d'une feuille métallique.

3. Capteur selon la revendication 1 ou 2, caractérisé en ce que le tapis aspirant (6) est un filet en nylon.

4. Capteur selon l'une des revendications 1 à 3, caractérisé en ce qu'entre les membranes individuelles (2) ou entre les moyens disposés sur celles-ci pour l'optimisation de la répartition de la solution aqueuse à mesurer et de la couche de protection (3), il est prévu un liquide d'étalonnage (5), de préférence une solution saline neutre.

5. Capteur selon l'une des revendications 1 à 4, caractérisé en ce que les membranes individuelles (2) contiennent une enzyme qui est sensible aux composants de matériaux à détecter.

6. Capteur selon l'une des revendications 1 à 5, caractérisé en ce que les membranes isolées (2) contiennent en plus de l'enzyme un médiateur.

7. Capteur selon l'une des revendications 1 à 6, caractérisé en ce que les membranes individuelles (2) sont sensibles à des matières physiologiques, de préférence le glucose dans le liquide corporel.

8. Capteur selon l'une des revendications 1 à 7, caractérisé en ce que les électrodes sont constituées par trois électrodes isolées entre elles, une électrode de travail, une électrode de contrôle et une électrode de référence, cette dernière étant de préférence réalisée en argent.
